# EUROPEAN PATENT SPECIFICATION

(11) **EP 4 319 884 B1**
(45) Date of publication and mention of the grant of the patent: **27.05.2026**
(21) Application number: 22719828.0
(22) Date of filing: 30.03.2022
(51) Int. Cl.: A61Q 5/00, A61Q 5/02, A61Q 5/12, A61K 8/35, A61K 8/37, A61K 8/49, A61K 8/44, A61Q 17/04, A61K 8/41

(54) **COSMETIC COMPOSITION COMPRISING HINOKITIOL**
KOSMETISCHE ZUSAMMENSETZUNG MIT HINOKITIOL
COMPOSITION COSMÉTIQUE COMPRENANT DE L'HINOKITIOL

(30) Priority: 07.04.2021 WO PCT/CN2021/085846; 18.05.2021 EP 21174223
(43) Date of publication of application: 14.02.2024
(73) Proprietor: Unilever IP Holdings B.V., 6708 WH Wageningen (NL); Unilever Global IP Limited, Wirral, Merseyside CH62 4ZD (GB)
(72) Inventor: MERRINGTON, James, 6708 WH Wageningen (NL); TANG, Xuezhi, 6708 WH Wageningen (NL)
(74) Representative: Unilever Patent Group
(86) International application number: PCT/EP2022/058359
(87) International publication number: WO 2022/214370

(56) References cited:
- WO-A1-2014/025413
- WO-A1-2018/172948
- US-A1- 2009 306 154
- US-B2- 8 382 856
- DATABASE GNPD [online] MINTEL; 11 May 2017 (2017-05-11), ANONYMOUS: "P5 Lotion", XP055860871, retrieved from https://www.gnpd.com/sinatra/recordpage/4818173/ Database accession no. 4818173
- DATABASE GNPD [online] MINTEL; 7 May 2020 (2020-05-07), ANONYMOUS: "Scalp Tonic", XP055860817, retrieved from https://www.gnpd.com/sinatra/recordpage/7565315/ Database accession no. 7565315
- ANONYMOUS: "Anti-dandruff hair composition", RESEARCH DISCLOSURE, KENNETH MASON PUBLICATIONS, HAMPSHIRE, UK, GB, vol. 429, no. 93, 1 January 2000 (2000-01-01), XP007125378, ISSN: 0374-4353
- DATABASE GNPD [online] MINTEL; 7 May 2020 (2020-05-07), ANONYMOUS: "Scalp Tonic", XP055860817, retrieved from www.gnpd.com Database accession no. 7565315
- DATABASE GNPD [online] MINTEL; 11 May 2017 (2017-05-11), ANONYMOUS: "P5 Lotion", XP055860871, retrieved from www.gnpd.com Database accession no. 4818173

## Description

### Field of the Invention

The present invention relates to cosmetic compositions, preferably hair care compositions. More particularly the invention relates to cosmetic compositions that comprise hinokitiol.

### Background of the Invention

Dandruff is a condition experienced by many people worldwide. The dandruff condition varies from mild symptoms such as flaking skin to severe inflammation and itchiness of the scalp. Malassezia yeasts, such as *Malassezia furfur,* are believed by some to be the main cause of dandruff and, whilst this might not represent the full scientific picture of the situation, Malassezia yeasts do appear to be closely associated with dandruff. Hence, the strategy conventionally used for the treatment of dandruff is the topical application of antifungals such as zinc pyrithione (ZnPTO), piroctone olamine, climbazole and ketoconazole which are normally delivered through a shampoo.

Hinokitiol is a tropolone originally isolated from the heart wood of T. plicata that has diverse biological activities, including antifungal, antibacterial, and antiproliferative properties. Hinokitiol has been used in oral, hair and skin care products. Generally, hinokitiol is dissolved in the base composition of a product which includes surfactants, water, polymers and colouring and fragrance ingredients. When a user applies the compositions to hair, oral or skin, compound of hinokitiol get deposited thereon. However, it is known that hinokitiol is light instable and the hinokitiol that is deposited may be not bioavailable as hinokitiol tends to react with UV radiation which leads to its destabilization.

It is thus an object of the present invention to enhance the stability of the hinokitiol bioactive.

US8382856B2 (FUJI FILM Corporation) relates to a non-oxidative dye having good light resistance, stainability and safety. Example 9 of D1 discloses a hair dye composition comprising hinokitiol and a UVA filter which is oxybenzone-3.

WO 2018172948A1 (RATTI KARAN) relates to a composition for lightening of skin and reducing hyper-pigmentation. It comprises an organic photo-protective agent and hinokitiol.

Non-patent literature "Anti-dandruff hair composition" Research Disclosure Kenneth publication, Hampshire, UK, GB, vol. 429, no. 93, 1 January 2000 ISSN:0374-4353 discloses an anti-dandruff hair composition comprising hinokitiol.

Mintel GNPD Record ID 7565315 "Scalp Tonic", May 2020 discloses an anti-hairloss functional tonic comprising hinokitiol and Camellia Sinensis Leaf Extract.

Mintel GNPD Record ID 4818173 "P5 Lotion", May 2017 discloses a leave-on treatment that fights dandruff, and it comprises hinokitiol and Benzyl Salicylate.

### Summary of the Invention

The present inventors have determined a new way of improving the problem of photo stability of a photolabile active which is hinokitiol. We have determined that photo stability of hinokitiol can be improved if hinokitiol and an organic UVA filter are coformulated into a composition at certain ratio.

In accordance with a first aspect, disclosed is a cosmetic composition comprising:
(i) hinokitiol;
(ii) an organic UVA filter selected from Butyl Methoxydibenzoylmethane, Bis-Ethylhexyloxyphenol Methoxyphenyl Triazine, Diethylamino Hydroxybenzoyl Hexyl Benzoate, Methylene Bis-benzotriazolyl Tetramethylbutylphenol, and a mixture thereof; and
(iii) a cosmetically acceptable carrier;
wherein the weight ratio of said UVA filter to hinokitiol is at least 1:20; wherein the composition is a shampoo composition

### Detailed Description of the Invention

For the avoidance of doubt, any feature of one aspect of the present invention may be utilized in any other aspect of the invention. The word "comprising" is intended to mean "including" but not necessarily "consisting of or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. As used herein, the indefinite article "a" or "an" and its corresponding definite article "the" means at least one, or one or more, unless specified otherwise. The various features of the present invention referred to in individual sections above apply, as appropriate, to other sections *mutatis mutandis.* Consequently, features specified in one section may be combined with features specified in other sections as appropriate. Any section headings are added for convenience only and are not intended to limit the disclosure in any way.

By 'a cosmetic composition' as used herein, is meant to include a composition for topical application to skin, hair and/or scalp of mammals, especially human beings. Such a composition is generally applied on to the desired topical surface of the body for a period of time from a few seconds to up to 24 hours. When the period of time of application is low say of the order of a few seconds to a few minutes after which the composition is rinsed off with water or wiped away, such a composition is known as a cleansing composition or a wash-off composition. On the other hand, When the composition is applied for longer period of time say from several minutes to up to 24 hours and washed off usually during the process of normal personal cleaning, such a composition is known as a leave-on composition. The composition as per the present invention includes any product applied to a human body for also improving appearance, cleansing, odor control or general aesthetics.

By "hair care composition" as used herein, is meant to include a composition for topical application to hair or scalp of mammals, especially humans. By topical is meant that the composition is applied to the external surface of the body. In the present invention this is achieved by applying the composition on the hair or scalp. Such a composition may be generally classified as leave-on or rinse off, and includes any product applied for improving the appearance, cleansing, odor control or general aesthetics of scalp and hair. The haircare composition of the present invention could be in the form of a liquid, lotion, cream, foam, scrub, gel, shampoo, conditioner, shower gel or bar. The haircare composition of the present invention is preferably a leave-on composition. Alternatively, the haircare composition of the present invention is a wash-off composition. Compositions for achieving the desired benefits by way of ingestion into the human body are excluded from the scope of the present invention.

### Hinokitiol

Hinokitiol (beta-thujaplicin) according to the present invention is a monoterpenoid that is cyclohepta-2,4,6-trien-1-one substituted by a hydroxy group at position 2 and an isopropyl group at position 4. The CAS number is 499-44-5 and the compound has the general formula (I) as below:

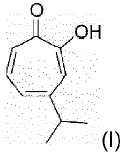

Amount of the hinokitiol in the composition of the invention would depend on the type of the cosmetic composition and the precise nature of other antimicrobial agents used. It is preferred that the composition comprises 0.01 to 10 wt% of said hinokitiol, more preferably 0.05 to 5 wt%, furthermore preferably 0.1 to 3 wt% by weight of the composition.

### Organic UVA filter

Hinokitiol is known to be light instable. It has been found that the UVA filter of the present invention unexpectedly enhances stability of hinokitiol. The composition comprises the UVA filter and hinokitiol in a weight ratio of at least 1:20, preferably at least 1:10. It is preferred that the composition comprises the UVA filter and hinokitiol in a weight ratio of from 1:20 to 10:1, preferably from 1:10 to 10:1, more preferably from 1:5 to 10:1, and most preferably from 1:2 to 5:1.

Organic UVA filters are individual compounds or mixtures that absorb ultraviolet A (UVA) light. The organic UVA filter as per the invention is selected from Butyl Methoxydibenzoylmethane, Bis-Ethylhexyloxyphenol Methoxyphenyl Triazine, Diethylamino Hydroxybenzoyl Hexyl Benzoate, Methylene Bis-benzotriazolyl Tetramethylbutylphenol, and a mixture thereof. Furthermore preferably the organic UV filter as per the invention is selected from Butyl Methoxydibenzoylmethane, Bis-Ethylhexyloxyphenol Methoxyphenyl Triazine, and a mixture thereof.

It is most preferred that the organic UVA filter as per the invention is Butyl Methoxydibenzoylmethane.

The amount of organic UVA filter incorporated into the compositions may depend on the amount of hinokitiol used. A preferred amount of organic UVA filter is from 0.1 to 15%, more preferably from 0.5 to 5%, furthermore preferably from 1 to 3% by weight of the total composition.

It is preferred that the composition of the invention additionally comprises an organic compound. Such organic compound can improve the solubility of organic UVA filter used in the composition. The solubility of the UVA filter in the organic compound is at least 5%. It can be selected from essential oils, emollient oils such as alcohols, fatty esters, ethers, triglycerides, carbonates and a mixture thereof. More preferably it can be selected from Ethylhexyl Methoxycinnamate, C12-15 Alkyl Benzoate, Dibutyl Adipate, Dicaprylyl Carbonate, Propylheptyl Caprylate, Cocoglycerides, Octocrylene, Homosalate, Ethylhexyl Salicylate, Bis-Ethylhexyl Hydroxydimethoxy Benzylmalonate, Diethylhexyl Syringylidene Malonate and Caprylic/ Capric Triglyceride, Ethyl Butylacetyl aminopropionate, Capric/Caprylic Triglyceride, Dicaprylyl Ether, Ethylhexyl Palmitate, Almond oil, Caprylyl-Caprylate/Caprate, Coco Caprylate, Isopropyl palmitate, Jojoba oil, and a mixture thereof. It is more preferred that the organic compound is Ethylhexyl Methoxycinnamate.

The amount of the additional organic compound in the composition may depend on the amount of organic UVA filter used. A preferred amount of such organic compound is from 0.2 to 30%, more preferably from 1 to 10%, furthermore preferably from 2 to 6% by weight of the total composition.

When Butyl Methoxydibenzoylmethane is used in the composition, it is preferred that the organic compound comprises Ethylhexyl Methoxycinnamate. The amount of Ethylhexyl Methoxycinnamate incorporated into the compositions may depend on the amount of Butyl Methoxydibenzoylmethane used. A preferred amount of Ethylhexyl Methoxycinnamate is from 0.2 to 30%, more preferably from 1 to 10%, furthermore preferably from 2 to 6% by weight of the total composition.

### Cosmetic composition

The composition of the invention preferably comprises a cosmetically acceptable carrier. The cosmetically acceptable carrier is such that the composition can be prepared as a shampoo.

### Hair care composition

The composition is a shampoo composition.

Antidandruff agents are compounds that are active against dandruff and are typically antimicrobial agents, preferably antifungal agents. Antidandruff agents typically display a minimum inhibitory concentration of about 50 mg/ml or less against *Malassezia.*

In addition to hinokitiol, the hair care composition in accordance with this invention may also comprise free additional antidandruff agent which is different from it. Whenever present, the hair care composition of the invention preferably comprises 0.1 to 5 wt%, more preferably 0.5 to 2% of the additional antidandruff agent.

The additional antidandruff agent is preferably selected from piroctone compound, azoles, selenium sulfide, salicylic acid and combinations thereof. Azoles include ketoconazole and climbazole, preferably climbazole. It is preferred that the additional antidandruff agent is piroctone compound, more preferably piroctone olamine.

It is preferable that the hair care composition also comprises a cationic polymer. Suitable cationic polymers may be homopolymers or be formed from two or more types of monomers. The molecular weight of the polymer will generally be between 5,000 and 10,000,000 g/mol, typically at least 10,000 g/mol and preferably from 100,000 to 2,000,000 g/mol. The polymers will have cationic nitrogen containing groups such as quaternary ammonium or protonated amino groups, or a mixture thereof. The cationic nitrogen containing group will generally be present as a substituent on a fraction of the total monomer units of the cationic polymer. Thus when the polymer is not a homopolymer it can contain spacer non-cationic monomer units. The ratio of the cationic to non-cationic monomer units is selected to give a polymer having a cationic charge density in the required range.

Suitable cationic polymers include, for example, copolymers of vinyl monomers having cationic amine or quaternary ammonium functionalities with water soluble spacer monomers such as (meth)acrylamide, alkyl and dialkyl (meth) acrylamides, alkyl (meth)acrylate, vinyl caprolactone and vinyl pyrrolidine. The alkyl and dialkyl substituted monomers preferably have C₁-C₇ alkyl groups, more preferably C₁-C₃ alkyl groups. Other suitable spacers include vinyl esters, vinyl alcohol, maleic anhydride, propylene glycol and ethylene glycol.

Preferably, the cationic polymer is a cationic polysaccharide polymer such as cationic guar, cationic starch, and cationic cellulose. Suitably, such cationic polysaccharide polymers have a molecular weight of from 100,000 g/mol to 2,300,000 g/mol, more preferably from 150,000 g/mol to 2,000,000 g/mol. Such cationic polysaccharide polymers preferably have a cationic charge density from 0.1 to 4 meq/g.

Cationic polysaccharide polymers suitable for use in compositions of this invention include those represented by the general formula:

A-O-[R¹-N⁺(R²)(R³)(R⁴)X⁻]

wherein: A is an anhydroglucose residual group, such as a starch or cellulose anhydroglucose residual. R¹ is an alkylene, oxyalkylene, polyoxyalkylene, or hydroxyalkylene group, or combination thereof. R², R³ and R⁴ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R², R³ and R⁴) is preferably about 20 or less, and X is an anionic counterion.

Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200.

Other suitable cationic polysaccharide polymers include quaternary nitrogen-containing cellulose ethers (e.g. as described in U.S. Patent 3,962,418) and copolymers of etherified cellulose and starch (e.g. as described in U.S. Patent 3,958,581).

A particularly preferred type of cationic polysaccharide polymer that can be used in compositions of the present invention is a cationic guar gum derivative, such as guar hydroxypropyltrimonium chloride (for example, commercially available from Solvay in their Jaguar trademark series or from Ashland in their N-Hance trademark series). Examples of such materials are Jaguar^{®} C-13S, Jaguar^{®} C-14S, Jaguar^{®} C-17, Jaguar^{®} Excel, Jaguar^{®} C-162, Jaguar^{®} C-500, Jaguar^{®} Optima, Jaguar^{®} LS, N-Hance^{™} BF17, N-Hance^{™} BF13 and N-Hance^{™} CCG45.

Mixtures of any of the above cationic polymers may be used. The cationic polymer preferably comprises cationic cellulose, cationic guar or mixtures thereof. Guar hydroxypropyltrimonium chloride is particularly preferred.

When used, the cationic polymer will generally be present in the hair care composition of the present invention in an amount of from 0.001 to 1% by weight of the hair care composition, more preferably from 0.01 to 0.5%, and most preferably from 0.03 to 0.3%, based on total weight of the hair care composition.

The hair care composition may additionally comprises a copolymer comprising acrylamidopropyltrimonium chloride. Preferably, the copolymer also comprises acrylamide in addition to the acrylamidopropyltrimonium chloride. Particularly preferred copolymer is a copolymer of acrylamidopropyltrimonium chloride and acrylamide.

The copolymer preferably has a charge density of from 1.0 to 3.0 meq per gram (meq/g), more preferably from 1.1 to 2.5 meq/g, more preferably still from 1.2 to 2.5 meq/g and most preferably from 1.3 to 2.5 meq/g. The copolymer preferably has a molecular weight of from 100,000 to 3,000,000 gram per mole (g/mol), more preferably from 500,000 to 2,800,000 g/mol, more preferably still from 800,000 to 2,500,000 g/mol, most preferably from 1,000,000 to 2,500,000 g/mol. An example of a suitable copolymer is commercially available from Ashland under the trade name N-Hance SP-100^{®}. N-Hance SP-100^{®} has a charge density of from 1.8 to 2.2 meq/g and a molecular weight of from 1,800,000 to 2,200,000 g/mol.

The hair care composition of the present invention preferably comprises the copolymer in an amount of from 0.001 to 2%, more preferably from 0.01 to 1%, even more preferably from 0.01 to 0.8% and most preferably from 0.05 to 0.5%, based on total weight of the hair care composition.

The hair care composition may additionally comprise a conditioning agent to provide conditioning benefit. Preferably, the hair care composition comprises discrete dispersed droplets of a water-insoluble conditioning agent, which has a mean droplet diameter (D_{3,2}) of less than 15 microns, preferably less than 10 microns, more preferably less than 5 microns, most preferably less than 3 microns. The mean droplet diameter (D_{3,2}) of a water-insoluble conditioning agent may be measured by means of a laser light scattering technique, for example using a 2600D Particle Sizer from Malvern Instruments.

The water-insoluble conditioning agent may include non-silicone conditioning agent comprising non-silicone oily or fatty materials such as hydrocarbon oils, fatty esters and mixtures thereof. Preferably, the water-insoluble conditioning agent is emulsified silicone oil.

Suitable silicones include polydiorganosiloxanes, in particular polydimethylsiloxanes which have the CTFA designation dimethicone. Also suitable for use in compositions of this invention (particularly shampoos and conditioners) are polydimethyl siloxanes having hydroxyl end groups, which have the CTFA designation dimethiconol. Also suitable for use in compositions of this invention are silicone gums having a slight degree of cross-linking, as are described for example in WO 96/31188. Preferably, the silicone oil comprises dimethicone, dimethiconol or a mixture thereof.

Suitable emulsified silicones for use in the hair care compositions of this invention are available as pre-formed silicone emulsions from suppliers of silicones such as Dow Corning and GE silicones. The use of such pre-formed silicone emulsion is preferred for ease of processing and control of silicone particle size. Such pre-formed silicone emulsions will typically additionally comprise a suitable emulsifier, and may be prepared by a chemical emulsification process such as emulsion polymerisation, or by mechanical emulsification using a high shear mixer. Examples of suitable pre-formed silicone emulsions include DC1785, DC1788, DC7128, all available from Dow Corning. These are emulsions of dimethiconol/dimethicone.

Another class of silicones which may be used are functionalized silicones such as amino functional silicones, meaning a silicone containing at least one primary, secondary or tertiary amine group, or a quaternary ammonium group. Examples of suitable amino functional silicones include polysiloxanes having the CTFA designation "amodimethicone."

The water-insoluble conditioning agent is generally present in hair care composition of this invention in an amount from 0.05 to 15%, preferably from 0.1 to 10%, more preferably from 0.5 to 8%, most preferably from 1 to 5%, based on total weight of the hair care composition.

In a preferred embodiment, the hair care composition is a shampoo. Thus in a preferred embodiment the composition comprises a cleansing surfactant. Non-limiting examples of suitable anionic cleansing surfactants are alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule. Typical anionic cleansing surfactants for use in compositions of the invention include, but not limited to, sodium oleyl succinate, ammonium lauryl sulphosuccinate, sodium lauryl sulphate, sodium lauryl ether sulphate, sodium lauryl ether sulphosuccinate, ammonium lauryl sulphate, sodium dodecylbenzene sulphonate, triethanolamine dodecylbenzene sulphonate, sodium cocoyl isethionate, sodium lauryl isethionate, lauryl ether carboxylic acid, sodium N-lauryl sarcosinate or mixtures thereof. Preferred anionic cleansing surfactants are the alkyl sulphates and alkyl ether sulphates. Preferred alkyl sulphates are C₈₋₁₈ alky sulphates, more preferably C₁₂₋₁₈ alkyl sulphates, preferably in the form of a salt with a solubilising cation such as sodium, potassium, ammonium or substituted ammonium. Examples are sodium lauryl sulphate (SLS) or sodium dodecyl sulphate (SDS). It is particularly preferred that the anionic cleansing surfactant is alkyl ether sulphate. Preferred alkyl ether sulphates are those having the formula: RO(CH₂CH₂O)ₙSO₃M; wherein R is an alkyl or alkenyl having from 8 to 18 (preferably 12 to 18) carbon atoms; n is a number having an average value of greater than at least 0.5, preferably between 1 and 3, more preferably between 2 and 3; and M is a solubilising cation such as sodium, potassium, ammonium or substituted ammonium. An example is sodium lauryl ether sulphate (SLES). Preferred alkyl ether sulphate is sodium lauryl ether sulphate having an average degree of ethoxylation of from 0.5 to 3, preferably from 1 to 3, more preferably from 2 to 3.

The anionic cleansing surfactants are typically present in hair care composition of the present invention at a level of from 0.5 to 45%, more preferably from 1.5 to 35% and most preferably from 5 to 20%, based on total weight of the hair care composition and including all ranges subsumed therein.

The composition as per the invention optionally and preferably additionally comprises co-surfactants such as amphoteric and zwitterionic surfactants to provide mildness to the composition. Suitable examples include alkyl amine oxides, alkyl betaines, alkyl amidopropyl betaines, alkyl sulphobetaines (sultaines), alkyl amphoacetates, alkyl amphopropionates, alkyl amidopropyl hydroxysultaines, wherein the alkyl group has from 8 to 19 carbon atoms. Preferably, the co-surfactant is a betaine surfactant. Cocamidopropyl betaine (CAPB) is particularly preferred.

When used, the co-surfactant typically makes up from 0.1 to 15%, more preferably from 0.5 to 8% and most preferably from 0.5 to 4% by weight of the hair care composition, based on total weight of the hair care composition and including all ranges subsumed therein.

Preferably the composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

The suspending agent is generally present in hair care composition of this invention in an amount of from 0.1 to 10%, more preferably from 0.2 to 6%, and most preferably from 0.3 to 4%, based on total weight of the hair care composition and including all ranges subsumed therein.

Preservatives may also be incorporated into the hair care composition of this invention to protect against the growth of potentially harmful microorganisms. Suitable traditional preservatives include alkyl esters of parahydroxybenzoic acid, hydantoin derivatives, propionate salts, and a variety of quaternary ammonium compounds. Illustrative yet non-limiting examples of the types of preservatives that may be used in this invention include, for examples, phenoxyethanol, sodium salicylate, methyl paraben, butyl paraben, propyl paraben, diazolidinyl urea, sodium dehydroacetate, benzyl alcohol, sodium benzoate, iodopropynyl butylcarbamate, caprylyl glycol, disodium EDTA or mixtures thereof. In an especially preferred embodiment, the preservative is sodium benzoate, phenoxyethanol, sodium salicylate or a mixture thereof. Preservatives are preferably employed in amounts ranging from 0.01 to 2% by weight of the hair care composition.

It is further preferred that the hair care composition of the invention comprises a cosmetic ingredient. Preferably the cosmetic ingredient is selected from the group consisting of a silicone, an antibacterial agent other than antidandruff agents, a foam booster, a perfume, encapsulates (for example encapsulated fragrance) a dye, a colouring agent, a pigment, a preservative, a thickener, a protein, a phosphate ester, a buffering agent, a pH adjusting agent, a pearlescer (for example; mica, titanium dioxide, titanium dioxide coated mica, ethylene glycol distearate (INCI glycol distearate)) and/or opacifier, a viscosity modifier, an emollient, a sunscreen, an emulsifier, a sensate active (for example menthol and menthol derivatives), vitamins, mineral oils, essential oils, lipids, natural actives, glycerin, natural hair nutrients such as botanical extracts, fruit extracts, sugar derivatives and amino acids, microcrystalline cellulose and mixtures thereof.

Preferably, the composition of the present invention includes from 0.01 to 20 wt% of the at least one cosmetic ingredient, more preferably from 0.05 to 10 wt%, still more preferably from 0.075 to 7.5 wt% and most preferably, from 0.1 to 5 wt% of the at least one cosmetic ingredient, by weight of the total composition.

The composition of the present invention may also comprise synergistic antimicrobial compounds that give synergistic antimicrobial benefit when used in combination with the antimicrobial active to enhance its properties and further inhibit the growth of bacterial/fungal. Non-limiting examples of these compounds include compounds having alcoholic groups (e.g. honokiol, magnolol or paeonol), Piperazines and a phenolic compound found in natural plant extract viz. thymol and terpeniol.

### Method and Use

The present invention also provides for a method of enhancing the stability of hinokitiol onto scalp comprising the step of applying the composition of the first aspect onto scalp surfaces of an individual followed by rinsing the surfaces with water. The method is preferably non-therapeutic in nature, more preferably cosmetic in nature.

The present invention also provides for a composition of the first aspect for use in preventing or alleviating the symptoms of dandruff. The use is preferably non-therapeutic in nature, preferably cosmetic in nature.

### Mode of Use

The composition of the invention is primarily intended for topical application to hair and skin, preferably hair and scalp.

When the composition is a shampoo, it is topically applied to the hair and then massaged into the hair and scalp. Then it is rinsed with water prior to drying the hair. A hair oil or hair serum, being leave-on hair care compositions, are left on for 1 to 10 hours after application before being washed off.

The invention will be further illustrated by the following, non-limiting Examples, in which all percentages quoted are by weight based on total weight unless otherwise stated.

The examples are intended to illustrate the invention and are not intended to limit the invention to those examples *per se.*

### Examples

### Example 1

This example demonstrated the UVA filter can affect the stability of hinokitiol in model sebum solution. All ingredients are expressed by weight percent of the total formulation, and as level of active ingredient.

The model sebum was prepared as shown in Table 1.

**Table 1**

| **Ingredient %** | **Model sebum** |
|---|---|
| Oleic acid | 8.00 |
| Isostearic acid | 4.00 |
| Tricaprin | 1.80 |
| Triolein (65%) | 28.20 |
| Triisostearin | 9.20 |
| Oleyl oleate | 11.90 |
| Myristyl myristate | 11.90 |
| Isostearyl isostearate | 6.00 |
| Squalene | 13.80 |
| Cholesterol oleate | 3.40 |
| Cholesterol | 1.70 |

Solution samples were prepared as shown in Table 2.

**Table 2**

| **Ingredient %** | **Samples** | | | | | | | | | |
|---|---|---|---|---|---|---|---|---|---|---|
| | **A** | **B** | **C** | **G** | **H** | **1** | **2** | **3** | **4** | **5** |
| Hinokitiol | 0.025 | 0.025 | 0.025 | 0.025 | 0.025 | 0.025 | 0.025 | 0.025 | 0.025 | 0.025 |
| Ethylhexyl Methoxycinnamate (UVB) | / | 0.05 | / | | | / | / | / | / | / |
| Octocrylene (UVB) | / | / | 0.05 | | | / | / | / | / | / |
| Terephthalylidene dicamphor sulfonic acid | | | | 0.05 | | | | | | |
| Disodium Phenyl Dibenzimidazole Tetrasulfonate | | | | | 0.05 | | | | | |
| Benzophenone-3 | / | / | / | | | 0.05 | / | / | / | / |
| Bis-Ethylhexyloxyphenol Methoxyphenyl Triazine | / | / | / | | | / | 0.05 | / | / | / |
| Butyl Methoxydibenzoylmethane | / | / | / | | | / | / | 0.05 | / | / |
| Diethylamino Hydroxybenzoyl Hexyl Benzoate | / | / | / | | | / | / | / | 0.05 | / |
| Methylene Bis-benzotriazolyl Tetramethylbutylphenol | / | / | / | | | / | / | / | / | 0.05 |
| Model sebum | To 100 | To 100 | To 100 | To 100 | To 100 | To 100 | To 100 | To 100 | To 100 | To 100 |

The compositions/ingredients shown in Table 2 above were subjected to an experiment that gives an estimate of the residual amount of hinokitiol after being exposed to UV light for a long time. Model sebum is used to mimic the environment after the hinokitiol is deposited on scalp. The % average residual amount of hinokitiol was measured using an invitro method which is described hereinafter.

### Method

1mL sample was put into a 2mL transparent glass vial. Put the vial in a chamber with UV light (UVA: 250 µw/cm²; UVB: 110 µw/cm²) for 4 hours' UV exposure. Take a 200µL aliquot of the UV-exposed sebum solution and dilute with 800µL methanol, and shake the mixture vigorously for 1 mins on Mini-shaker. After the sebum layer settles down from methanol solution to the bottom, take 200µL supernatant for UPLC analysis to quantify the amount of hinokitiol left after UV exposure, denoted as A_{left}. Dilute another model sebum solution of hinokitiol and UV filter without UV exposure by using methanol as above and quantify the amount of hinokitiol in the solution by UPLC method, denoted as Aₜₒₜₐₗ. Then the hinokitol residual after UV exposure could be calculated based on the following equation: Hinokitiol residual (%) = A_{left} / Aₜₒₜₐₗ * 100%.

The results were summarized as shown in Table 3.

**Table 3**

| **Samples** | **A** | **B** | **C** | **G** | **H** | **1** | **2** | **3** | **4** | **5** |
|---|---|---|---|---|---|---|---|---|---|---|
| %average residual of hinokitiol | 0 | 0 | 0 | 0.8 | 6.5 | 15.20 | 74.90 | 64.50 | 63.40 | 65.40 |

The data contained in Table 3 clearly indicates that stability of hinokitiol improves remarkedly when the organic UVA filter in accordance with this invention is used (Compositions 2-5) during the experiments, as compared to corresponding samples outside the invention (Compositions A without UV filter, Composition B and C with an organic UVB filter, Composition G, H and 1 with other UVA filters).

### Example 2

This example demonstrated that the weight ratio of UV filter to hinokitiol can affect the stability of hinokitiol in model sebum solution. All ingredients are expressed by weight percent of the total formulation, and as level of active ingredient.

Solution samples were prepared as shown in Table 4.

**Table 4**

| **Ingredient %** | **Samples** | | | | |
|---|---|---|---|---|---|
| | **D** | **6** | **7** | **8** | **9** |
| Hinokitiol | 0.025 | 0.025 | 0.025 | 0.025 | 0.025 |
| Butyl Methoxydibenzoylmethane | 0.0005 | 0.00125 | 0.0025 | 0.005 | 0.05 |
| Model sebum | To 100 | To 100 | To 100 | To 100 | To 100 |
| Weight ratio of UVA filter to hinokitiol | 1:50 | 1:20 | 1:10 | 1:5 | 2:1 |

The compositions/ingredients shown in Table 4 above were subjected to an experiment that gives an estimate of the residual amount of hinokitiol after being exposed to UV light for a long time. Model sebum is used to mimic the environment after the hinokitiol is deposited on scalp. The % average residual amount of hinokitiol was measured using the same invitro method which is described in Example 1.

The results were summarized as shown in Table 5.

**Table 5**

| **Samples** | **D** | **6** | **7** | **8** | **9** |
|---|---|---|---|---|---|
| %average residual of hinokitiol | 0 | 7.90 | 17.30 | 46.40 | 64.50 |

The data contained in Table 5 clearly indicates that stability of hinokitiol can be improved when the weight ratio of organic UVA filter to hinokitiol is at least 1:20. Samples 8 and 9 comprising a higher weight ratio of organic UVA filter to hinokitiol showed significantly better stability of hinokitiol compared to samples 6 and 7.

### Example 3

This example demonstrated that the UVA filter can affect the stability of hinokitiol after application of shampoo compositions on scalp. All ingredients are expressed by weight percent of the total formulation, and as level of active ingredient.

Shampoo samples were prepared as shown in Table 6

**Table 6**

| **Ingredient %** | **Samples** | | | | |
|---|---|---|---|---|---|
| | **E** | **10** | **11** | **12** | **13** |
| Sodium Laureth Sulfate 1EO | 13.00 | 13.00 | 13.00 | 13.00 | 13.00 |
| Cocamidopropyl Betaine | 1.00 | 1.00 | 1.00 | 1.00 | 1.00 |
| Guar Hydroxypropyltrimonium Chloride | 0.20 | 0.20 | 0.20 | 0.20 | 0.20 |
| Hinokitiol | 1.00 | 1.00 | 1.00 | 1.00 | 1.00 |
| Butyl Methoxydibenzoylmethane | / | 2.00 | / | / | / |
| Bis-Ethylhexyloxyphenol Methoxyphenyl Triazine | / | / | 2.00 | / | / |
| Diethylamino Hydroxybenzoyl Hexyl Benzoate | / | / | / | 2.00 | / |
| Methylene Bis-benzotriazolyl Tetramethylbutylphenol | / | / | / | / | 2.00 |
| Sodium Salicylate | 0.30 | 0.30 | 0.30 | 0.30 | 0.30 |
| Phenoxyethanol | 0.50 | 0.50 | 0.50 | 0.50 | 0.50 |
| Citric Acid | 0.05 | 0.05 | 0.05 | 0.05 | 0.05 |
| Sodium Chloride | 1.20 | 1.20 | 1.20 | 1.20 | 1.20 |
| Water | To 100 | To 100 | To 100 | To 100 | To 100 |

### Methods

### - Deposition protocol

A piece of VITROSKIN (5.2 cm×5.2 cm,) was fixed on a plastic ring to get a VITROSKIN plate (Area about 11 cm²). 0.2 g hinokitiol shampoo sample was added to the plate. 1.8 mL DI water was added and mixed with shampoo on the VITROSKIN plate via a Teflon stirring rod for 60 seconds. Then the shampoo slurry was removed via pipette. For rinse, 4mL DI water was added to the plate and stirred with the Teflon rod for 30 seconds, and all the liquid was removed from the plate. The rinse step was repeated once. The shampoo treated VITROSKIN plate was dried naturally with light protection. And then dry VITROSKIN plate was cut down from the plastic holder and immersed into 7 mL methanol in a 15 mL centrifuge tube with ultrasonic treatment for 10 mins. 1 mL of methanol solution was passed through a 0.2 µm PTFE filter, and the filtrate was transferred into a sample vial for Liquid Chromatography analysis to get the amount of deposited hinokitiol, denoted as D₀.

### - UV treatment protocol

After one piece of VITROSKIN plate was washed with hinokitiol shampoo sample as above and dried naturally in dark, 120µL model sebum was applied onto the VITROSKIN plate and dispersed evenly. The plate was placed in a chamber with UV light (UVA: 250 µw/cm2; UVB: 110 µw/cm2) for 1hr's UV exposure. Then the VITROSKIN plate was cut down from the plastic holder and immersed into 7 mL methanol in a 15 mL centrifuge tube with ultrasonic treatment for 10 mins. 4 mL of methanol solution was concentrated up to dryness. The residual was re-dissolved with 0.2 mL methanol. The 0.2 mL methanol solution was passed through a 0.2 µm PTFE filter, and the filtrate was transferred into a sample vial for Liquid Chromatography analysis to get the amount of left hinokitiol after UV treatment, denoted as D.

Then the hinokitiol residual after deposition and UV treatment could be calculated based on the following equation: hinokitiol residual (%) = D/D₀ * 100%
The results were summarized as shown in Table 7.

**Table 7**

| **Samples** | **E** | **10** | **11** | **12** | **13** |
|---|---|---|---|---|---|
| %average residual of hinokitiol | 2.1 | 9.6 | 5.6 | 3.6 | 3.5 |

The data contained in Table 7 clearly indicates that stability of hinokitiol after application on scalp improves markedly when shampoo samples comprising the organic UVA filter in accordance with this invention.

### Example 4

This example demonstrated the incorporation of Ethylhexyl Methoxycinnamate in the shampoo comprising hinokitol and Butyl Methoxydibenzoylmethane can affect the stability of hinokitiol after application of shampoo compositions on scalp. All ingredients are expressed by weight percent of the total formulation, and as level of active ingredient.

Shampoo samples were prepared as shown in Table 8

**Table 8**

| **Ingredient** | **Samples** | | | |
|---|---|---|---|---|
| | **E** | **F** | **10** | **14** |
| Sodium Laureth Sulfate 1EO | 13.00 | 13.00 | 13.00 | 13.00 |
| Cocamidopropyl Betaine | 1.00 | 1.00 | 1.00 | 1.00 |
| Guar Hydroxypropyltrimonium Chloride | 0.20 | 0.20 | 0.20 | 0.20 |
| Hinokitiol | 1.00 | 1.00 | 1.00 | 1.00 |
| Butyl Methoxydibenzoylmethane | / | / | 2.00 | 2.00 |
| Ethylhexyl Methoxycinnamate | / | 4.00 | / | 4.00 |
| Sodium Salicylate | 0.30 | 0.30 | 0.30 | 0.30 |
| Phenoxyethanol | 0.50 | 0.50 | 0.50 | 0.50 |
| Citric Acid | 0.05 | 0.05 | 0.05 | 0.05 |
| Sodium Chloride | 1.20 | 1.20 | 1.20 | 1.20 |
| Water | To 100 | To 100 | To 100 | To 100 |

### Methods

The % average residual amount of hinokitiol after application of shampoo on scalp was measured using the same invitro method disclosed in Example 3.

The results were summarized as shown in Table 9.

**Table 9**

| **Samples** | **E** | **F** | **10** | **14** |
|---|---|---|---|---|
| %average residual of hinokitiol | 2.1 | 2.2 | 9.6 | 21.4 |

The data contained in Table 9 clearly indicates that the incorporation of an organic compound for the organic UVA filter in the compositions in accordance with this invention can improve the stability of hinokitiol.

### Example 5

This example demonstrated the UVA filter outside the scope of the present invention cannot improve the stability of hinokitiol in model sebum solution. All ingredients are expressed by weight percent of the total formulation, and as level of active ingredient.

## Claims

1. A cosmetic composition comprising:
(i) hinokitiol;
(ii) an organic UVA filter selected from Butyl Methoxydibenzoylmethane, Bis-Ethylhexyloxyphenol Methoxyphenyl Triazine, Diethylamino Hydroxybenzoyl Hexyl Benzoate, Methylene Bis-benzotriazolyl Tetramethylbutylphenol, and a mixture thereof; and
(iii) a cosmetically acceptable carrier;
wherein the weight ratio of said UVA filter to hinokitiol is at least 1:20; wherein the composition is a shampoo composition.

2. A cosmetic composition as claimed in claim 1 wherein said composition additionally comprises an organic compound.

3. A cosmetic composition as claimed in claim 1 or 2 wherein the organic compound is selected from Ethylhexyl Methoxycinnamate, C12-15 Alkyl Benzoate, Dibutyl Adipate, Dicaprylyl Carbonate, Propylheptyl Caprylate, Cocoglycerides, Octocrylene, Homosalate, Ethylhexyl Salicylate, Bis-Ethylhexyl Hydroxydimethoxy Benzylmalonate, Diethylhexyl Syringylidene Malonate and Caprylic/ Capric Triglyceride, Ethyl Butylacetyl aminopropionate, Capric/Caprylic Triglyceride, Dicaprylyl Ether, Ethylhexyl Palmitate, almond oil, Caprylyl-Caprylate/Caprate, Coco Caprylate, Isopropyl palmitate, jojoba oil, and a mixture thereof, preferably Ethylhexyl Methoxycinnamate.

4. A cosmetic composition as claimed in any of claims 1 to 3 wherein the weight ratio of said UVA filter to hinokitiol is from 1:20 to 10:1.

5. A cosmetic composition as claimed in any of claims 1 to 4 wherein amount of said hinokitiol is from 0.01 to 10% by weight of the composition.

6. A cosmetic composition as claimed in any of claims 1 to 5 wherein amount of said organic UVA filter is from 0.1 to 15% by weight of the composition.

7. A cosmetic composition as claimed in any of claims 1 to 6 additionally comprising a cleansing surfactant, preferably sodium lauryl ether sulphate.

8. A cosmetic composition as claimed in any of claims 1 to 7 wherein said composition comprising a conditioning agent, preferably silicone oil, more preferably dimethicone, dimethiconol or a mixture thereof.

9. A cosmetic composition as claimed in any of claims 1 to 8 wherein said composition additionally comprises a cationic polymer, preferably selected from cellulose, cationic guar or mixtures thereof.

10. A cosmetic composition as claimed in claim 9 wherein said composition comprising a cationic guar polymer, preferably guar hydroxypropyltrimonium chloride.

11. A method of enhancing the stability of hinokitiol onto scalp comprising the step of applying the composition as claimed in any of claims 1 to 10 onto scalp surfaces of an individual followed by rinsing the surfaces with water.

12. A composition as claimed in any of claims 1 to 10 for use in preventing or alleviating the symptoms of dandruff.

## Patentansprüche

1. Kosmetische Zusammensetzung, umfassend:
(i) Hinokitiol;
(ii) einen organischen UVA-Filter, ausgewählt unter Butylmethoxydibenzoylmethan, Bis-Ethylhexyloxyphenolmethoxyphenyltriazin, Diethylaminohydroxybenzoylhexylbenzoat, Methylen-bisbenzotriazolyltetramethylbutylphenol und einer Mischung davon; und
(iii) einen kosmetisch verträglichen Träger;
wobei das Gewichtsverhältnis des UVA-Filters zu Hinokitiol mindestens 1:20 beträgt;
wobei die Zusammensetzung eine Shampoo-Zusammensetzung ist.

2. Kosmetische Zusammensetzung, wie im Anspruch 1 beansprucht, wobei die Zusammensetzung zusätzlich eine organische Verbindung umfasst.

3. Kosmetische Zusammensetzung, wie im Anspruch 1 oder 2 beansprucht, wobei die organische Verbindung ausgewählt ist unter Ethylhexylmethoxycinnamat, C₁₂₋₁₅-Alkylbenzoat, Dibutyladipat, Dicaprylylcarbonat, Propylheptylcaprylat, Cocoglyceriden, Octocrylen, Homosalat, Ethylhexylsalicylat, Bis-Ethylhexylhydroxydimethoxybenzylmalonat, Diethylhexylsyringylidenmalonat und Capryl-/Capric-Triglycerid, Ethylbutylacetylaminopropionat, Capric-/Capryl-Triglycerid, Dicaprylylether, Ethylhexylpalmitat, Mandelöl, Caprylylcaprylat/caprat, Kokos-Caprylat, Isopropylpalmitat, Jojobaöl und eine Mischung davon, vorzugsweise Ethylhexylmethoxycinnamat.

4. Kosmetische Zusammensetzung, wie in einem der Ansprüche 1 bis 3 beansprucht, wobei das Gewichtsverhältnis des UVA-Filters zu Hinokitiol 1:20 bis 10:1 beträgt.

5. Kosmetische Zusammensetzung, wie in einem der Ansprüche 1 bis 4 beansprucht, wobei die Menge an Hinokitiol 0,01 bis 10 Gew.-% der Zusammensetzung beträgt.

6. Kosmetische Zusammensetzung, wie in einem der Ansprüche 1 bis 5 beansprucht, wobei die Menge des organischen UVA-Filters 0,1 bis 15 Gew.-% der Zusammensetzung beträgt.

7. Kosmetische Zusammensetzung, wie in einem der Ansprüche 1 bis 6 beansprucht, die zusätzlich ein reinigendes Tensid enthält, vorzugsweise Natriumlaurylethersulfat.

8. Kosmetische Zusammensetzung, wie in einem der Ansprüche 1 bis 7 beansprucht, wobei die Zusammensetzung ein Konditionierungsmittel, vorzugsweise Silikonöl, bevorzugter Dimethicone, Dimethiconol oder eine Mischung davon, umfasst.

9. Kosmetische Zusammensetzung, wie in einem der Ansprüche 1 bis 8 beansprucht, wobei die Zusammensetzung zusätzlich ein kationisches Polymer umfasst, das vorzugsweise unter Cellulose, kationischem Guar oder Mischungen davon ausgewählt ist.

10. Kosmetische Zusammensetzung, wie im Anspruch 9 beansprucht, wobei die Zusammensetzung ein kationisches Guarpolymer, vorzugsweise Guarhydroxypropyltrimoniumchlorid, umfasst.

11. Verfahren zur Verbesserung der Stabilität von Hinokitiol auf der Kopfhaut, umfassend den Schritt des Aufbringens der Zusammensetzung, wie in einem der Ansprüche 1 bis 10 beansprucht, auf die Kopfhautoberflächen einer Person, gefolgt vom Abspülen der Oberflächen mit Wasser.

12. Zusammensetzung, wie in einem der Ansprüche 1 bis 10 beansprucht, zur Verwendung bei der Vorbeugung oder Linderung der Symptome von Schuppen.

## Revendications

1. Composition cosmétique comprenant :
(i) du hinokitiol ;
(ii) un filtre UVA organique choisi parmi le butylméthoxydibenzoylméthane, la biséthylhexyloxyphénolméthoxyphényltriazine, le benzoate de diéthylaminohydroxybenzoylhexyle, le méthylènebis-benzotriazolyl-tétraméthylbutylphénol, et leurs mélanges ; et
(iii) un véhicule acceptable en cosmétique ;
dans laquelle le rapport en poids dudit filtre UVA au hinokitiol est d'au moins 1/20 ; dans laquelle la composition est une composition de shampooing.

2. Composition cosmétique selon la revendication 1, dans laquelle ladite composition comprend de plus un composé organique.

3. Composition cosmétique selon la revendication 1 ou 2, dans laquelle le composé organique est choisi parmi le méthoxycinnnamate d'éthylhexyle, un benzoate d'alkyle en C12-15, l'adipate de dibutyle, le carbonate de dicaprylyle, le caprylate de propylheptyle, les glycérides dérivés du coprah, l'octocrylène, l'homosalate, le salicylate d'éthylhexyle, le malonate de bis-éthylhexylhydroxydiméthoxybenzyle, le malonate de diéthylhexylsyringylidène et le triglycéride caprylique/caprique, l'aminopropionate d'éthylbutylacétyle, le triglycéride caprique/caprylique, le dicaprylyléther, le palmitate d'éthylhexyle, l'huile d'amande, le caprylate/caprate de caprylyle, le caprylate dérivé du coprah, le palmitate d'isopropyle, l'huile de jojoba, et leurs mélanges, de préférence le méthoxycinnamate d'éthylhexyle.

4. Composition cosmétique selon l'une quelconque des revendications 1 à 3, dans laquelle le rapport en poids dudit filtre UVA au hinokitiol est de 1/20 à 10/1.

5. Composition cosmétique selon l'une quelconque des revendications 1 à 4, dans laquelle la quantité dudit hinokitiol est de 0,01 à 10 % en poids de la composition.

6. Composition cosmétique selon l'une quelconque des revendications 1 à 5, dans laquelle la quantité dudit filtre UVA organique est de 0,1 à 15 % en poids de la composition.

7. Composition cosmétique selon l'une quelconque des revendications 1 à 6, comprenant de plus un tensioactif nettoyant, de préférence le lauryléthersulfate de sodium.

8. Composition cosmétique selon l'une quelconque des revendications 1 à 7, dans laquelle ladite composition comprend un agent de traitement, de préférence une huile siliconée, mieux encore la diméthicone, le diméthiconol ou un de leurs mélanges.

9. Composition cosmétique selon l'une quelconque des revendications 1 à 8, dans laquelle ladite composition comprend de plus un polymère cationique, de préférence choisi parmi la cellulose, le guar cationique et leurs mélanges.

10. Composition cosmétique selon la revendication 9, dans laquelle ladite composition comprend un polymère de guar cationique, de préférence le chlorure d'hydroxypropyltrimonium-guar.

11. Procédé pour accroître la stabilité du hinokitiol sur le cuir chevelu, comprenant l'étape d'application de la composition de l'une quelconque des revendications 1 à 10 sur des surfaces du cuir chevelu d'un individu, suivie d'un rinçage à l'eau des surfaces.

12. Composition selon l'une quelconque des revendications 1 à 10, pour une utilisation dans la prévention ou l'atténuation des symptômes de pellicules.
